# EUROPEAN PATENT SPECIFICATION

(11) **EP 0 568 656 B1**
(45) Date of publication and mention of the grant of the patent: **20.05.1998**
(21) Application number: 92919159.1
(22) Date of filing: 26.08.1992
(51) Int. Cl.: A61B 1/07, A61B 1/00, A61B 5/00

(54) **ILLUMINATED BOUGIE FOR LAPAROSCOPIC IMPLANTATION OF ANTI-REFLUX PROSTHESIS**
BELEUCHTETE BOUGIE ZUR LAPAROSKOPISCHEN IMPLANTATION VON ANTI-REFLUX-PROTHESEN
BOUGIE ECLAIREE UTILISEE POUR L'IMPLANTATION PAR LAPAROSCOPIE D'UNE PROTHESE ANTI-REFLUX

(30) Priority: 07.10.1991 US 772332
(43) Date of publication of application: 10.11.1993
(73) Proprietor: Angelchik, Jean Pierre, Phoenix, Arizona 85012 (US)
(72) Inventor: Angelchik, Jean Pierre, Phoenix, Arizona 85012 (US)
(74) Representative: Brereton, Paul Arthur
(86) International application number: US9207356
(87) International publication number: WO9306769

(56) References cited:
- EP-A- 0 138 089
- CH-A- 208 643
- DE-U- 8 900 120
- US-A- 1 246 340
- US-A- 1 704 764
- US-A- 2 482 971
- US-A- 2 797 683
- US-A- 5 006 106
- Laryngoscope 96, Sept. 1986, page 1040: "New instrument: illuminated bougie for use in cervical esophageal surgery" F.E. Lejeune, M.L. Cheney.

## Description

This invention relates to an illuminated bougie for insertion into the body.

More particularly, the invention concerns an instrument for laparascopically implanting a prosthesis for controlling gastro esophageal reflux.

Methods and apparatus for the prevention of gastro esophageal reflux are described in my prior United States Patents Nos 3,875,928, issued April 8, 1975, and 4,271,828, issued June 9, 1981. An alternate method, generally employing a similar prosthesis is also described in my prior United States Patent No 4,271,827, issued June 9, 1981. A method for laparoscopically implanting such prostheses is disclosed in my prior United States Patent No 5,006,106 issued November 9, 1991.

The principal object of the present invention is to provide an illuminated bougie for use in practicing the method disclosed in my issued US Patent No 5,006,106 and in other diagnostic, therapeutic or surgical procedures.

A bougie according to the pre-characterising part of Claim 1 is known from the publication entitled Laryngoscope, Volume 96, page 1040 (1986) which describes a device consisting of a hollow tapered esophageal bougie. An elliptical portal was formed in the distal end of the bougie. A fibre optic bundle, the terminal end of which was cut at a 40° angle, was then inserted into the lumen of the bougie such that the terminal end of the bundle was positioned in the portal. The terminal end of the bundle was then cemented to fix it in the portal. Thus, the bougie described in this article could only transilluminate a very small portion of the esophagus which was directly adjacent the oval portal at the distal end of the bougie. By contrast, as will appear more fully below, the bougie which I have invented can transilluminate a substantial length of the esophagus.

Schellenberg, US 1,704,764, disclosed a hollow perforate colonic exploration device formed of translucent rubber, into which a hot-filament electric lamp could be temporarily inserted, to facilitate taking a picture of the walls of the colons and intestines. Aiken, US 2,797,683, disclosed a rigid bronchoscope formed of a plastic material which transmits light by internal reflection, to be emitted by the leading (distal) end of the device, in a manner similar to the Laryngoscope article cited above.

Swiss Patent No 208643 discloses an illuminated speculum for inspecting body cavities.

A bougie according to the present invention as defined according to the characterising part of Claim 1.

In the drawings:
Figure 1 depicts an illuminated bougie embodying the present invention; and
Figure 2 depicts a procedure for laparoscopic implantation of an anti reflux prosthesis, utilizing the bougie of Figure 1.

The drawings are presented for the purpose of illustrating the practice of my invention, so as to enable those skilled in the art to understand and practice it, but are not intended as limitations upon the scope of the invention. Further, the drawings illustrate the best mode which I presently contemplate for carrying out my invention, again without intending to limit the scope thereof. In these drawings, like reference characters depict the same elements in the several view.

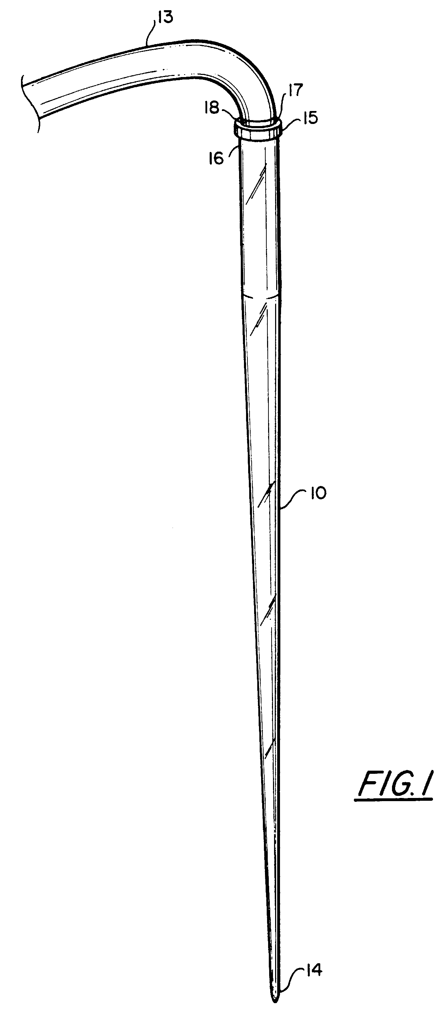

Turning now to the drawings, Figure 1 depicts the presently preferred embodiment of the invention, consisting of a bougie 10 formed entirely of light-transmitting material. The upper proximal portion A is cylindrically shaped and the remainder is tapered towards the distal end 14. The bougie 10 is imperforate and is preferably solid. A socket 15 is formed in the proximal end 16 of the bougie 10 to receive the light-transmitting terminal end 17 of a fibre optics bundle 13. The socket 15 and collar 18 cooperate to attach the terminal end 17 of the fibre optic bundle 13 to the proximal end 16 of the bougie 10. In the embodiment depicted in Figure 1 light from the terminal end 17 of the fibre optic bundle 13 is transmitted throughout the entire length of the bougie 10 for transillumination of adjacent tissue.

The shape and dimensions of the bougie can be varied to suit the end use of the device. Thus, for upper laryngeal and esophagus surgery, i.e., Zencker's diverticulum, Barrett's esophagus resections, thoracoscopy, e.g., surgery of the mediastinum, surgery of the abdomen when it is important to see the stomach, esophagus and/or for laparoscopic surgery for ulcer disease, vagotomy, for morbid obesity, for reflux esophagitis and for placement of the prosthesis in accordance with my '106 Patent, the length of the bougie 10 is suitably 20 cm for adults and 15 cm for children. The diameter is suitably 36-40 French for adults and 18-26 French for children. (1 French = ¹/₃ mm.)

In the preferred embodiment the bougie 10 is formed of moulded silicone plastic or similar plastic having a hardness of approximately 50 durometer. Micrometalic particles can be dispersed in the plastic to improve lateral diffusion of the light. Polishing the external surface of the bougie causes light to be transmitted further toward the distal end before it is diffused laterally. However, when the esophagus or tissue closes on the bougie, the bougie surface-air interface is altered, causing lateral diffusion of the light along the length contacted by the espohagus.

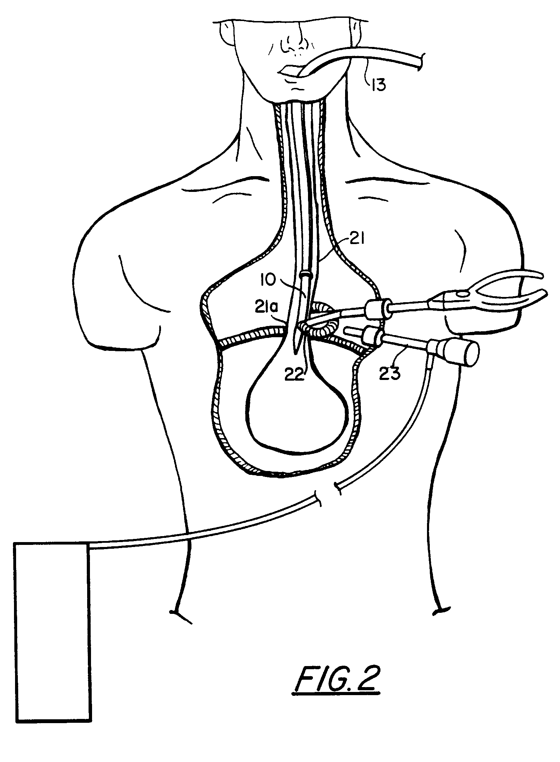

A typical laparoscopic procedure involving use of the bougie of my invention is depicted in Fig. 2. The procedure is facilitated by illuminating by the esophagus 21 by means of the bougie 10 which is inserted intra-orally. The wall 21a of the distal esophagus is sufficiently thin to transmit a substantial portion of the light emitted by the bougie 10, facilitating viewing the esophagus and placement of the prosthesis 22, viewing the procedure through a laparoscopic optical viewing device 23.

Since no heat is transmitted through the bougie, it can be left in place during the entire course of the procedure, even several hours, if necessary, without burning the body tissue it contacts, because the light source is a fiber optic bundle attached to a long fiber optic cord and is many feet away from the light source, rather than an incandescent filament inside the bougie.

Having now described my invention is such terms as to enable those skilled in the art to understand and practice it without undue experimentation and, having disclosed the best mode I presently contemplate for carrying out my invention, I claim:

## Claims

1. An illuminated bougie for illuminating the body, the bougie (10) being elongate and dimensioned for insertion into the body, the bougie (10) having socket means (15) for attaching the light transmitting end (17) of a fibre optic bundle (13), characterised in that the socket means (15) is formed in the proximal end (16) of the bougie (10) and the entire length of the bougie (10) is formed of a flexible material which transmits and diffuses light to transilluminate adjacent body tissue along the said length.

2. A bougie according to Claim 1 characterised in that a proximal portion (A) of the bougie is cylindrically shaped.

3. A bougie according to Claim 1 or 2 characterised in that the bougie (10) is tapered towards the distal end (14).

4. A bougie according to any of the preceding claims characterised in that the bougie (10) is unperforate.

5. A bougie according to any of the preceding claims characterised in that the bougie (10) is solid.

6. A bougie according to any of the preceding claims characterised in that the bougie (10) is formed of plastics material.

7. A bougie according to Claim 6 characterised in that the bougie is of silicone plastics.

8. A bougie according to Claim 6 or 7 characterised in that the micrometallic particles are dispersed in the plastics material to improve lateral diffusion of light.

9. A bougie according to any of Claims 6 to 8 characterised in that the plastics material has a hardness of approximately 50 durometer.

## Patentansprüche

1. Beleuchtete Bougie zur Beleuchtung des Körpers, wobei die Bougie (10) ein längliches Teil ist und eine Abmessung zur Einführung in den Körper aufweist, wobei die Bougie (10) eine Sockeleinrichtung (15) zum Befestigen des lichtdurchlässigen Endes (17) eines optischen Faserbündels (13) aufweist, dadurch gekennzeichnet, daß die Sockeleinrichtung (15) im proximalen Ende (16) der Bougie (10) gebildet ist und die Bougie (10) über die gesamte Länge aus einem flexiblen Material gebildet ist, das Licht zum Durchleuchten von benachbartem Körpergewebe entlang dieser Länge überträgt und zerstreut.

2. Bougie nach Anspruch 1, dadurch gekennzeichnet, daß ein proximaler Abschnitt (A) der Bougie zylindrisch geformt ist.

3. Bougie nach Anspruch 1 oder 2, dadurch gekennzeichnet, daß die Bougie (10) in Richtung auf das distale Ende (14) spitz zuläuft.

4. Bougie nach einem der vorangehenden Ansprüche, dadurch gekennzeichnet, daß die Bougie (10) perforationsfrei ist.

5. Bougie nach einem der vorangehenden Ansprüche, dadurch gekennzeichnet, daß die Bougie (10) massiv ist.

6. Bougie nach einem der vorangehenden Ansprüche, dadurch gekennzeichnet, daß die Bougie (10) aus Kunststoffmaterial gebildet ist.

7. Bougie nach Anspruch 6, dadurch gekennzeichnet, daß die Bougie aus Silikon-Kunststoff besteht.

8. Bougie nach Anspruch 6 oder 7, dadurch gekennzeichnet, daß die mikrometallischen Partikel in dem Kunststoffmaterial verteilt sind, um die seitliche Lichtdiffusion zu verbessern.

9. Bougie nach einem der Ansprüche 6 bis 8, dadurch gekennzeichnet, daß das Kunststoffmaterial eine Härte von ungefähr 50 Durometer bzw. Härtegraden aufweist.

## Revendications

1. Bougie éclairée destinée à éclairer le corps, la bougie (10) étant allongée et dimensionnée pour pouvoir être insérée dans le corps, la bougie (10) possédant des moyens (15) formant cavité pour fixer l'extrémité (17) transmettant la lumière d'un faisceau de fibres optiques (13), caractérisée en ce que les moyens (15) formant cavité sont formés dans l'extrémité proximale (16) de la bougie (10) et toute la longueur de la bougie (10) est formée d'une matière flexible, qui transmet et diffuse la lumière pour transilluminer les tissus du corps sur ladite longueur.

2. Bougie selon la revendication 1, caractérisée en ce qu'une portion proximale (A) de la bougie est de forme cylindrique.

3. Bougie selon la revendication 1 ou 2, caractérisée en ce que la bougie (10) s'amincit vers son extrémité distale (14).

4. Bougie selon une des revendications précédentes, caractérisée en ce que la bougie (10) est non perforée.

5. Bougie selon une quelconque des revendications précédentes, caractérisée en ce que la bougie (10) est pleine.

6. Bougie selon une quelconque des revendications précédentes, caractérisée en ce que la bougie (10) est formée en une matière plastique.

7. Bougie selon la revendication 6, caractérisée en ce que la bougie est faite d'une matière plastique de silicone.

8. Bougie selon la revendication 6 ou 7, caractérisée en ce que les particules micrométalliques sont dispersées dans la matière plastique pour améliorer la diffusion latérale de la lumière.

9. Bougie selon une quelconque des revendications 6 à 8, caractérisée en ce que la matière plastique possède une dureté d'environ 50 au duromètre.
